# EUROPÄISCHE PATENTSCHRIFT

(11) **EP 0 966 979 B1**
(45) Veröffentlichungstag und Bekanntmachung des Hinweises auf die Patenterteilung: **08.03.2006**
(21) Anmeldenummer: 99111383.8
(22) Anmeldetag: 11.06.1999
(51) Int. Cl.: A61L 29/00, A61F 2/06, A61L 31/02

(54) **Implantierbare, bioresorbierbare Gefässwandstütze, insbesondere Koronarstent**
Implantable bioresorbable support for the vascular walls, in particular coronary stent
Support pour la paroi des vaisseaux implantable et biodégradable, notamment un extenseur coronaire

(30) Priorität: 25.06.1998 DE 19828245; 10.12.1998 DE 19856983
(43) Veröffentlichungstag der Anmeldung: 29.12.1999
(73) Patentinhaber: Biotronik AG, 8180 Bülach (CH)
(72) Erfinder: Bolz, Armin, Dr., 76356 Weingarten (DE); Popp, Thomas, 90443 Nürnberg (DE)
(74) Vertreter: Lindner-Vogt, Karin L.

(56) Entgegenhaltungen:
- WO-A-99/03515
- DE-A- 19 731 021
- US-A- 3 687 135

## Beschreibung

Die Erfindung betrifft eine implantierbare, bioresorbierbare Gefäßwandstütze, insbesondere einen Koronarstent.

Zum Hintergrund der Erfindung ist festzuhalten, daß sogenannte "Gefäßwandstützen" oder - wie sie im Fachjargon genannt werden - "Stents" zur Therapie von Stenosen, also krankhaft verengten Passägen eines ;Koronargefäßes eingesetzt werden. Dazu wird ein solcher Koronarstent mittels eines Katheters transvenös in den Körper eingeführt und durch das Gefäßsystem an die Problemstelle im Herzen vorgeschoben. In diesem Zustand des Einführens und Vorschiebens darf der Stent für eine ausreichende Beweglichkeit einen Außendurchmesser von lediglich etwa 1 mm aufweisen. Ist die stenotische Koronargefäß-Passage erreicht, wird der Stent dauerhaft aufgeweitet, um die Stenose zu beheben. Dazu wird der Stent mittels des Katheters, auf dem er eingeführt wurde, unter plastischer Verformung bis auf einen Durchmesser von etwa 4 mm radial aufgeweitet. Zu diesem Zweck ist der Katheter als Ballonkatheter ausgeführt, bei dem der den Stent tragende Längsabschnitt durch Anlegen eines Überdrucks mittels Kochsalzlösung ähnlich einem Luftballon dilatiert wird.

Herkömmliche implantierte Stents bestehen aus einem für den medizinischen Einsatz geeigneten Metallmaterial, das gegebenenfalls zur Vermeidung thrombotischer Probleme mit einer antikoagulative wirkenden Beschichtung versehen sein kann. Nachteil bei solchen dauerhaft implantierten Stents ist die nachweislich auftretende permanente Reizung des den Stent umgebenden Gewebes, da er die durch den Herzschlag verursachten Biegebewegungen des von ihm gestützten Koronargefäßes aufgrund seiner Steifigkeit nicht nachvollzieht.

Ferner ist darauf hinzuweisen, daß zur Aufweitung einer Stenose die Unterstützung durch den Stent in der Regel nur über einen Zeitraum von einigen Monaten erforderlich ist. Danach bliebe die von der Stenose betroffene Gefäßpartie auch ohne Unterstützung offen.

Zur Behebung der vorgenannten Probleme wurde bereits vorgeschlagen, Stents aus bioresorbierbaren Materialien herzustellen, die im Körper im Verlauf weniger Monate zersetzt werden. Ein Herstellungsverfahren für solche bioresorbierbaren Koronarstents ist z. B. aus der DE 195 39 449 A1 bekannt. Dort wird aus einer viskosen Lösung von Poly-Beta-Hydroxy-Buttersäure als bioresorbierbaren Polymermaterial in einem Lösungsmittel durch sukzessives, schichtweises Aufbringen der Polymerlösung auf einen Positiv-Formkern in mehreren Schritten durch Abscheiden des Polymermaterials unter Abdampfen des Lösungsmittels und unter zumindest teilweiser Anlösung der vorher abgeschiedenen Schicht ein in seiner Polymerstruktur homogener Stenterrohling aufgebaut. Dieser wird vom Positiv-Formkem abgezogen und zur Endformgebung des Stents nachbearbeitet.

Bioresorbierbare Koronarstents aus Polymermaterialien weisen zwar die gewünschte biologische Resorbierbarkeit und Körperverträglichkeit auf. Probleme bereiten jedoch die oft unzureichenden mechanischen Eigenschaften, wie beispielsweise eine mangelnde plastische Verformbarkeit dieses Stents. Dies führt bei der Dilatation auf immerhin den 4fachen Durchmesser zu einer Rißbildung mit der Folge einer herabgesetzten mechanischen Stabilität und zu einer hohen Rückverformung. Letzteres bedeutet, daß für einen Enddurchmesser von 4 mm die maximale Aufweitung deutlich darüber liegen muß. Dies wiederum führt zu einer weiter erhöhten Rißbildung mit entsprechender Destabilisierung des Stents.

Zur Lösung der vorstehenden Problematik sieht die Erfindung laut Patentanspruch 1 vor, dass die Gefäßwandstütze aus einer sich im Körper ohne schädliche Auswirkungen auf den Implantat-Träger zersetzenden Metall-Materialkombination besteht, als elektrochemisches Lokal-Element ausgelegt ist, das einerseits durch den Körper der Gefäßstütze aus einem im Wesentlichen reiner ersten Metall und andererseits durch eine damit in Kontakt stehende, zu einer Kontaktspannung führenden Lokalelektrode aus einem zweiten Metall gebildet ist. Die Metall-Materialkombination ist dabei so auszulegen, daß sich das Material der Gefäßwandstütze mit einer bestimmten Zersetzungsrate und ohne die Produktion von körperschädlichen Zersetzungsprodukten auflöst. Eine derartige Gefäßwandstütze vereint also die vorteilhaften mechanischen Eigenschaften von Metall-Stents mit der Bioresorbierbarkeit von Stents auf Polymer-Basis.

Erfindungsgemäss ist die Metall-Materialkombination als elektrochemisches Lokal-Element ausgelegt. Dieses besteht einerseits aus einem den Körper der Gefäßwandstütze bildenden, im wesentlichen reinen ersten Metall und andererseits aus einer damit in Kontakt stehenden Lokalelektrode aus einem zweiten Metall. Letztere bildet mit dem Stützenkörper ein Lokal-Element, bei dem das Potential des Stützenkörpers entsprechend der elektrochemischen Spannungsreihe verschoben ist. Die entstehende Kontaktspannung verursacht den Korrosionsprozeß des Stentkörpers. Die Korrosionsrate und damit die Zersetzungszeit des Stents kann über die Größe der Kontaktfläche zwischen dem korrodierbaren Stentkörper und der damit verbundenen Lokalelektrode bzw. über die Auswahl des Partnerelementes selbst gesteuert werden.

Gemäß zweier alternativer Ausführungsformen kann das Partnerelement als Beschichtung auf dem Stützenkörper oder als eigenständiges, an dem Stützenkörper angebrachtes, beispielsweise daran angeschweißtes Metallteil ausgebildet sein. Im letzteren Fall können die Lokalelektroden in einer typischen Doppelfunktion auch als Röntgenmarker dienen.

Im folgenden wird die Erfindung an einem bevorzugten Ausführungsbeispiel näher erläutert:

### Beispiel

Bei einem bioresorbierbaren Metallstent besteht der Stützenkörper aus reinem Zink, das sich - wie galvanische Versuche zeigen - bei externen Strömen von einigen mA ohne Gasentwicklung und ohne Oxidbildung auflöst, wie dies für korrodierbare Metallstents anzustreben ist.

Zur Erzeugung eines solchen "externen" Stromes ist nun ein Lokal-Element z.B. in Form einer Goldelektrode galvanisch oder durch Laserschweißen auf dem Stent angebracht und führt als Lokalelektrode zusammen mit dem Stützenkörper zu einer Kontaktspannung, da die Goldelektrode näherungsweise das Potential der Zinkelektrode annimmt und das Potential der Zinkelektrode geringfügig positiver wird. Es folgt ein entsprechender Strom, der zur aktiven Zinkauflösung führt. An der Goldelektrode fließen entsprechende kathodische Ströme. Der Austauschstrom insgesamt wird wegen der geringen kathodischen Ströme der Goldelektrode von der Fläche der Goldelektrode bestimmt. Über diesen Mechanismus kann die Korrosionsrate also über die Fläche des Lokal-Elementes eingestellt werden.

Wie Versuche gezeigt haben, stellt sich bei einem Gold-Lokal-Element auf Zink ein Austauschstrom nach wenigen Minuten ein, der über mehrere Tage konstant bleibt. Insoweit ist eine konstante Korrosionsrate erzielbar, wobei beispielsweise ein 10 mg schwerer Stent bei 10 µA Korrosionsstrom sich in zirka 30 bis 40 Tagen auflösen wird.

## Patentansprüche

1. Implantierbare, bioresorbierbare Gefäßwandstütze, insbesondere Koronarstent, **dadurch gekennzeichnet, daß** die Gefäßwandstütze aus einer sich im Körper ohne schädliche Auswirkungen auf den Implantat-Träger zersetzenden Metall-Materialkombination besteht, die als elektrochemisches Lokal-Element ausgelegt ist, das einerseits durch den Körper der Gefäßwandstütze aus einem im wesentlichen reinen ersten Metall und andererseits durch eine damit in Kontakt stehende, zu einer Kontaktspannung führenden Lokalelektrode aus einem zweiten Metall gebildet ist.

2. Gefäßwandstütze nach Anspruch 1, **dadurch gekennzeichnet, daß** die Lokalelektrode als Beschichtung auf dem Stützenkörper ausgebildet ist.

3. Gefäßwandstütze nach Anspruch 1, **dadurch gekennzeichnet, daß** die Lokalelektrode als an dem Stützenkörper angebrachtes, insbesondere daran angeschweißtes Metallteil ausgebildet ist.

4. Gefäßwandstütze nach einem der Ansprüche 1 bis 3, **dadurch gekennzeichnet, daß** der Stützenkörper aus Zink besteht.

5. Gefäßwandstütze nach einem der Ansprüche 1 bis 4, **dadurch gekennzeichnet, daß** die Lokalelektrode aus einem Edelmetall, insbesondere Gold, Platin oder Iridium besteht.

6. Gefäßwandstütze nach Anspruch 2, **dadurch gekennzeichnet, daß** die die Lokalelektrode bildende Beschichtung durch Galvanisierung oder Sputtern aufgebracht ist.

## Claims

1. An implantable bioresorbable vascular stent, in particular a coronary stent, **characterised in that** the vascular stent consists of a metal-material combination which decomposes in the body without any damaging effects on the implant carrier, wherein said metal-material combination is an electrochemical local element which is formed on the one hand by the body of the vascular stent, made from an essentially pure first metal, and on the other hand by a local electrode made from a second metal, which is in contact with the body of the vascular stent, wherein said local electrode carries a contact voltage.

2. The vascular stent according to claim 1, **characterised in that** the local electrode is a coating on the stent body.

3. The vascular stent according to claim 1, **characterised in that** the local electrode is a metal part that is attached to, in particular welded to, the stent body.

4. The vascular stent according to any one of claims 1 to 3, **characterised in that** the stent body comprises zinc.

5. The vascular stent according to any one of claims 1 to 4, **characterised in that** the local electrode comprises a noble metal, in particular gold, platinum or iridium.

6. The vascular stent according to claim 2, **characterised in that** the coating which forms the local electrode has been applied by galvanising or sputtering.

## Revendications

1. Soutien de paroi de vaisseau, implantable et biorésorbable, notamment stent coronaire, **caractérisé en ce que** le soutien de paroi coronaire est composé d'une combinaison métal-matière se décomposant dans le corps sans effets nocifs sur le soutien d'implant et se présentant sous forme d'un élément électrochimique local qui est constitué d'une part par le corps du soutien de paroi coronaire fait d'un premier métal sensiblement pur et d'autre part d'une électrode locale en contact avec celui-ci et provoquant une tension de contact, faite d'un deuxième métal.

2. Soutien de paroi de vaisseau selon la revendication 1, **caractérisé en ce que** l'électrode locale se présente sous forme d'un revêtement du corps de soutien.

3. Soutien de paroi de vaisseau selon la revendication 1, **caractérisé en ce que** l'électrode locale se présente sous forme d'une pièce métallique fixée au corps de soutien, notamment y soudée.

4. Soutien de paroi de vaisseau selon une des revendications 1 à 3, **caractérisé en ce que** le corps de soutien est composé de zinc.

5. Soutien de paroi de vaisseau selon une des revendications 1 à 4, **caractérisé en ce que** l'électrode locale est composée d'un métal rare, notamment de l'or, du platine ou de l'iridium.

6. Soutien de paroi de vaisseau selon la revendication 2, **caractérisé en ce que** le revêtement formant l'électrode locale est appliqué par galvanisation ou pulvérisation cathodique.
